# EUROPÄISCHE PATENTSCHRIFT

(11) **EP 3 177 358 B1**
(45) Veröffentlichungstag und Bekanntmachung des Hinweises auf die Patenterteilung: **11.11.2020**
(21) Anmeldenummer: 15748176.3
(22) Anmeldetag: 04.08.2015
(51) Int. Cl.: A61M 39/10, F16L 13/11, F16L 13/12, F16L 21/00, F16L 25/00, F16L 55/17, A61M 5/36

(54) **VERFAHREN ZUR ERHÖHUNG DER DICHTIGKEIT EINES MECHANISCHEN KONNEKTORS**
METHOD OF INCREASING AIR-TIGHTNESS OF A CONNECTOR
PROCÉDÉ DE COLMATAGE HERMÉTIQUE D'UN RACCORD

(30) Priorität: 05.08.2014 DE 102014011674
(43) Veröffentlichungstag der Anmeldung: 14.06.2017
(73) Patentinhaber: Fresenius Medical Care Deutschland GmbH, 61352 Bad Homburg (DE)
(72) Erfinder: WOJKE, Ralf, 61348 Bad Homburg (DE); WIENEKE, Paul, 48149 Münster (DE)
(74) Vertreter: Herrmann, Uwe
(86) Internationale Anmeldenummer: PCT/EP2015/001606
(87) Internationale Veröffentlichungsnummer: WO 2016/020055

(56) Entgegenhaltungen:
- EP-A1- 2 258 972
- DE-A1- 10 213 814
- DE-U1- 29 908 172
- GB-A- 2 383 828
- GB-A- 2 439 091
- US-A- 4 830 914
- US-A- 5 030 487
- US-A- 5 908 211
- US-A1- 2008 017 269
- "Sicherheitsdatenblatt Locher-Paste 2000", , 13. Januar 2011 (2011-01-13), XP055220930, Internet Gefunden im Internet: URL:http://www.viscotex.ch/index.php?TPL=1 0181 [gefunden am 2015-10-14]

## Beschreibung

Die Erfindung betrifft ein Verfahren zur Erhöhung der Dichtigkeit eines mechanischen Konnektors einer extrakorporalen Blutbehandlungsmaschine sowie eine extrakorporale Blutbehandlungseinheit.

Mikroblasen sind Gasblasen mit einem Durchmesser von wenigen µm, die aufgrund ihrer geringen Größe in der Regel nicht mehr sichtbar sind. Sie entstehen an verschiedenen Stellen und unter verschiedenen Bedingungen in extrakorporalen Blutkreisläufen, unter anderem durch Austreten von blutlöslichen Gasen oder Lufteintritt an kleinsten Undichtigkeiten im Unterdruckbereich des extrakorporalen Kreislaufs, und haften an der inneren Oberfläche des extrakorporalen Schlauchsystems oder der Blutbehandlungseinheit an. Ihre Auswirkungen, wenn sie losgelöst und in den menschlichen Körper infundiert werden, sind stärker als bisher vermutet. Beispielsweise wurden Mikroblasen in lebenswichtigen Organen wie Lunge, Herz und Hirn von Dialysepatienten nachgewiesen.

Es gibt Hinweise darauf, dass beispielsweise die Luer-Lock-Verbindung zwischen arterieller Kanüle und dem arteriellen Schlauch, eine Verbindung von zwei konischen Hartplastikteilen, die sich im Unterdruckbereich der Blutpumpe befindet, nicht immer luftdicht ist und geringe Mengen Luft in den Blutschlauch gesaugt werden. In einem Luer-Lock Konnektor wird ein harter Kunststoff gegen einen harten Kunststoff gepresst, wobei der abgedichtete Bereich einer solchen flächigen Pressung auf einem Konus begrenzt ist. Untersuchungen haben bestätigt, dass im Unterdruckbereich Mikroblasenanzahl und -volumen stromabwärts eines Luer-Lock Konnektors deutlich größer sind als stromaufwärts eines Luer-Lock Konnektors.

Mikroblasen können aufgrund ihrer geringen Größe und ihres geringen Auftriebes nur eingeschränkt in der venösen Kammer abgeschieden und von dem vorgeschriebenen Schutzsystem zur Vermeidung von Luftinfusion nur bedingt erkannt werden.

Dokument GB2383828 beschreibt die Verbindung zweier Luer Elemente aber löst nicht das genannte Problem.

Der Erfindung liegt die Aufgabe zu Grunde, die Zahl an Mikroblasen in extrakorporalen Blutkreisläufen bzw. die Infusion von Mikroblasen in den Körper des Patienten zu verringern.

Die Erfindung ist definiert nach Ansprüchen 1 und 8.

Eine Flüssigkeit mit Viskositätswerten entsprechend Ansprüchen 1 oder 8 ist besonders geeignet für eine einfache Handhabung in der Auftragung und eine wirksame Abdichtung. Insbesondere soll die viskose Flüssigkeit blutverträglich sein, d.h. ein Eintrag einer kleinen Menge der Flüssigkeit in das Blut eines Patienten soll unschädlich sein.

Die Verbindungsteile und insbesondere die Kontaktflächen der Verbindungsteile können in einer Ausführungsform aus Hartplastik gefertigt sein.

In einer Ausführungsform handelt es sich bei dem mechanischen Konnektor um einen Luer-Lock-Anschluss. Dabei handelt es sich um einen genormten Konnektor für die Medizintechnik, wobei ein Verbindungsteil des Konnektors einen männlichen Konus und das andere Verbindungspaar einen weiblichen Konus aufweist. Um die Verbindungen gegen Lösen optimal zu sichern, weist der Luer-Lock-Anschluss neben den Koni ferner ein System zum Verschrauben der Verbindungsteile auf, wobei ein Außengewinde an dem Verbindungsteil, in dem sich der weibliche Konus befindet, in ein Innengewinde eingreift, das an dem Verbindungsteil mit dem männlichen Konus sitzt.

Vor dem Zusammenführen der Verbindungsteile wird nur die Dichtfläche eines Verbindungsteils mit der viskosen Flüssigkeit benetzt. Beispielsweise kann vorgesehen sein, dass nur die Mantelfläche des männlichen Konus mit der viskosen Flüssigkeit benetzt wird. Dies ist zur Erstellung einer luftundurchlässigen Verbindung ausreichend und die äußere Mantelfläche des männlichen Konus ist besser zugänglich als die innere Mantelfläche des weiblichen Konus.

In einer Ausführungsform handelt es sich bei dem medizinischen Gerät um eine extrakorporale Blutbehandlungsmaschine. Bei der extrakorporalen Blutbehandlungsmaschine kann es sich um ein Dialysegerät handeln. Ferner kann es sich bei der extrakorporalen Blutbehandlungsmaschine beispielsweise um ein Ultrafiltrationsgerät oder eine Herz-Lungen-Maschine handeln. Der Einsatz des erfindungsgemäßen Verfahrens ist auch in anderen medizinischen Geräten denkbar. Beispielsweise ist die Anwendung auch in der endoskopischen Bauchchirurgie bzw. Gynäkologie denkbar, wobei mit Hilfe von Gasen eine artifizielle Operationshöhle geschaffen werden kann. Hier wäre eine gasundurchlässige Verbindung sehr sinnvoll, um die eingebrachte Menge an Gas zu begrenzen.

In einer Ausführungsform verbindet der Konnektor, an dem das Verfahren durchgeführt wird, einen flüssigkeitsführenden Kreislauf mit einer Zu- oder Ableitung. Beispielsweise kann es sich bei dem flüssigkeitsführenden Kreislauf um einen extrakorporalen Blutkreislauf handeln. Dieser Kreislauf ist typischerweise als Disposable ausgebildet, wobei das Disposable verschiedene Schnittstellen aufweist, die mit Hilfe eines Konnektors mit maschinenseitigen Leitungen, Kanülen, etc. in Verbindung stehen. Beispiele für Zu- und Ableitungen im Sinne dieser Ausführungsform umfassen den arteriellen Port, eine Prädilutionsleitung, eine Postdilutionsleitung, eine Heparinleitung, einen Port zur Blutabnahme, einen Port zur Medikamentengabe, einen Ablauf oder den venösen Port. Insbesondere kann vorgesehen sein, dass der Konnektor, an dem das Verfahren durchgeführt wird, eine arterielle Kanüle mit der arteriellen Leitung eines extrakorporalen Blutkreislaufs verbindet (arterieller Port).

In einer Ausführungsform umfasst der flüssigkeitsführende Kreislauf mindestens eine Pumpe, und das Verfahren wird bei einem Konnektor durchgeführt, der auf der Saugseite dieser mindestens einen Pumpe angeordnet ist. Insbesondere auf der Saugseite der Pumpe kann Luft in den Kreislauf gelangen, da gegenüber der Umgebung ein Unterdruck herrscht. Beispielsweise handelt es sich bei dem flüssigkeitsführenden Kreislauf um einen extrakorporalen Blutkreislauf und das Verfahren wird bei einem Konnektor durchgeführt, der auf der Saugseite der Blutpumpe angeordnet ist.

In einer Ausführungsform handelt es sich bei der Hülle um eine flexible Hülle. Mit Hilfe einer flexiblen Hülle kann eine dichtere Verbindung erreicht werden als mit einem starren Protektor, da sich die Hülle an die Außenoberfläche des Konnektors anschmiegen kann. Ferner ist eine flexible Hülle kostengünstig herzustellen und kann einfach nach dem Zusammenführen der Verbindungsteile über den Konnektor gestreift werden. Es kann vorgesehen sein, dass die Hülle so über den Konnektor übergestreift wird, dass sie den Bereich des Verbindungsspaltes vollständig überdeckt.

In einer Ausführungsform handelt es sich bei der Hülle um ein flexibles Schlauchstück oder einen Ring. Diese Formen ermöglichen es, die Hülle nach dem Zusammenführen der Verbindungsteile in einfacher Weise über den Konnektor zu stülpen.

In einer Ausführungsform ist die Hülle aus einem Silikonelastomer gefertigt. Dieses Material hat sich aufgrund der guten Eigenschaften bezüglich Elastizität, Biokompatibilität und Dichtigkeit als besonders geeignet erwiesen.

Die Erfindung betrifft ferner ein medizinisches Gerät umfassend einen mechanischen Konnektor, der ein Paar an Verbindungsteilen aufweist, die korrespondierende Dichtflächen aufweisen, wobei die Dichtflächen bei geschlossenem Konnektor zumindest abschnittsweise mit einer viskosen Flüssigkeit benetzt sind, und/oder wobei der Verbindungsspalt zwischen den Verbindungsteilen des geschlossenen Konnektors mit einer Hülle abgedeckt ist.

Der geschlossene Konnektor bezeichnet den Zustand des Konnektors nach dem Zusammenführen der Verbindungsteile. Bei dem medizinischen Gerät kann es sich um eine extrakorporale Blutbehandlungsmaschine handeln. Bei der extrakorporalen Blutbehandlungsmaschine kann es sich um ein Dialysegerät handeln. Ferner kann es sich bei der extrakorporalen Blutbehandlungsmaschine beispielsweise um ein Ultrafiltrationsgerät oder eine Herz-Lungen-Maschine handeln. Ferner ist denkbar, dass es sich bei dem medizinischen Gerät beispielsweise um ein Instrument der endoskopischen Bauchchirurgie bzw. Gynäkologie handelt. Auch weitere medizinische Geräte, an denen eine gasundurchlässige Verbindung zweier Verbindungsteile sinnvoll ist, werden von der vorliegenden Erfindung umfasst.

In einer Ausführungsform weist ein Verbindungsteil einen männlichen Konus und ein Verbindungsteil einen weiblichen Konus auf, wobei wenigstens ein Abschnitt der äußeren Mantelfläche des männlichen Konus und wenigstens ein Abschnitt der inneren Mantelfläche des weiblichen Konus die Dichtflächen bilden. In einer Ausführungsform handelt es sich bei dem mechanischen Konnektor um einen Luer-Lock-Anschluss.

In einer Ausführungsform verbindet der Konnektor einen flüssigkeitsführenden Kreislauf, insbesondere einen extrakorporalen Blutkreislauf mit einer Zu- oder Ableitung. Beispielsweise kann es sich bei dem flüssigkeitsführenden Kreislauf um einen extrakorporalen Blutkreislauf handeln. Dieser Kreislauf ist typischerweise als Disposable ausgebildet, wobei das Disposable verschiedene Schnittstellen aufweist, die mit Hilfe eines Konnektors mit maschinenseitigen Leitungen, Kanülen, etc. in Verbindung stehen. Beispiele für Zu- und Ableitungen im Sinne dieser Ausführungsform umfassen den arteriellen Port, eine Prädilutionsleitung, eine Postdilutionsleitung, eine Heparinleitung, einen Port zur Blutabnahme, einen Port zur Medikamentengabe, einen Ablauf oder den venösen Port. In einer Ausführungsform umfasst der flüssigkeitsführende Kreislauf mindestens eine Pumpe und der Konnektor ist der auf der Saugseite dieser mindestens einen Pumpe angeordnet. Beispielsweise handelt es sich bei dem flüssigkeitsführenden Kreislauf um einen extrakorporalen Blutkreislauf und der Konnektor ist auf der Saugseite der Blutpumpe angeordnet. Insbesondere kann vorgesehen sein, dass der Konnektor eine arterielle Kanüle mit der arteriellen Leitung eines extrakorporalen Blutkreislaufs verbindet (arterieller Port).

In einer Ausführungsform handelt es sich bei der Hülle um eine flexible Hülle, wobei vorzugsweise vorgesehen ist, dass diese Hülle den Bereich des Verbindungsspaltes vollständig überdeckt. Bei der Hülle kann es sich um ein flexibles Schlauchstück oder einen Ring handeln. Die Hülle kann aus einem Silikonelastomer gefertigt sein.

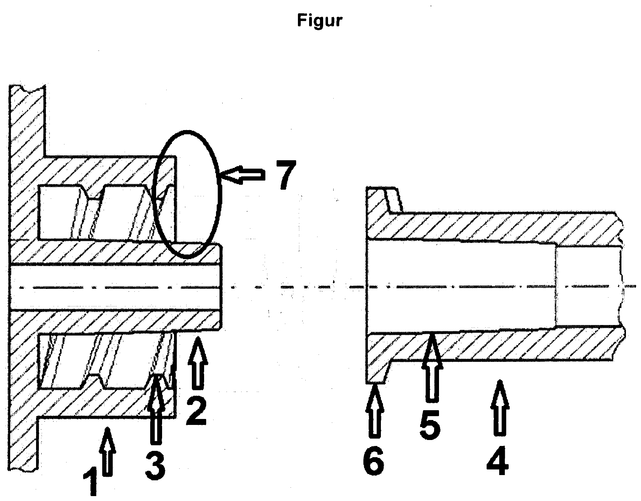

Weitere Einzelheiten und Vorteile ergeben sich aus den nachfolgend beschriebenen Figuren und Ausführungsbeispielen. Die einzige Figur zeigt eine Darstellung eines Luer-Lock Konnektors im Querschnitt.

Der Konnektor weist ein erstes Verbindungsteil 1 mit einem männlichen Konus 2 und einem Innengewinde 3 auf. Das zweite Verbindungsteil 4 weist einen weiblichen Konus 5 und einen Vorsprung 6 auf, der in das Innengewinde eingreift. Beim Zusammenführen der Verbindungsteile 1 und 4 werden diese verschraubt und die äußere Mantelfläche des männlichen Konus 2 und die innere Mantelfläche des weiblichen Konus 5 aneinandergepresst.

Erfindungsgemäß ist vorgesehen, dass vor dem Zusammenführen der beiden Verbindungsteile 1 und 4 die äußere Mantelfläche des männlichen Konus 2 mit einer viskosen, sterilen und bioverträglichen Flüssigkeit benetzt wird.

Alternativ oder zusätzlich ist erfindungsgemäß vorgesehen, dass nach dem Zusammenführen der Verbindungsteile 1 und 4 ein Schlauchstück aus einem flexiblen Silikonelastomer über den Konnektor gestreift wird. Der Innendurchmesser des flexiblen Schlauchstücks bzw. Ringes wird etwas kleiner gewählt als der Außendurchmesser des Konnektors, um einen luftdichten Abschluss zu erreichen. Das Schlauchstück soll insbesondere den Verbindungsspalt vollständig überdecken, der zwischen den beiden Verbindungsteilen nach deren Zusammenführen an der mit dem Bezugszeichen 7 gekennzeichneten Stelle entsteht.

## Patentansprüche

1. Verfahren zur Erhöhung der Dichtigkeit eines mechanischen Konnektors eines medizinischen Geräts, insbesondere einer Dialysemaschine, wobei
der mechanische Konnektor ein Paar an Verbindungsteilen (1, 4) aufweist, die korrespondierende Dichtflächen aufweisen,
ein Verbindungsteil (1) einen männlichen Konus (2) und das andere Verbindungsteil (4) einen weiblichen Konus (5) aufweist, wobei wenigstens ein Abschnitt der äußeren Mantelfläche des männlichen Konus (2) und wenigstens ein Abschnitt der inneren Mantelfläche des weiblichen Konus (5) die Dichtflächen bilden, und
ein Zusammenführen der Verbindungsteile (1, 4) umfasst, dass der Kontakt der beiden Dichtflächen erhalten bleibt und die Dichtflächen mit einem gewissen Anpressdruck durch Verschrauben oder Verrasten aufeinanderliegen,
**dadurch gekennzeichnet, dass**
vor dem Zusammenführen der Verbindungsteile (1, 4) eine der beiden Dichtflächen mit einer viskosen Flüssigkeit benetzt wird,
die viskose Flüssigkeit eine dynamische Viskosität von zwischen 10² und 10⁵ mPa·s bei 25°C aufweist, und
die viskose Flüssigkeit steril und biokompatibel ist.

2. Verfahren nach einem der vorhergehenden Ansprüche, **dadurch gekennzeichnet, dass** es sich bei dem mechanischen Konnektor um einen Luer-Lock-Anschluss handelt.

3. Verfahren nach einem der vorhergehenden Ansprüche, **dadurch gekennzeichnet, dass** vor dem Zusammenführen der Verbindungsteile (1, 4) nur die Dichtfläche eines Verbindungsteils (1), vorzugsweise nur die Mantelfläche des männlichen Konus (2) mit der viskosen Flüssigkeit benetzt wird.

4. Verfahren nach einem der vorhergehenden Ansprüche, **dadurch gekennzeichnet, dass** der Verbindungsspalt zwischen den Verbindungsteilen (1, 4) nach deren Zusammenführen mit einer Hülle abgedeckt wird

5. Verfahren nach Anspruch 4, **dadurch gekennzeichnet, dass** es sich bei der Hülle um eine flexible Hülle handelt, wobei vorzugsweise vorgesehen ist, dass diese Hülle so über den Konnektor übergestreift wird, dass sie den Bereich des Verbindungsspaltes vollständig überdeckt.

6. Verfahren nach einem der vorhergehenden Ansprüche 4 oder 5, **dadurch gekennzeichnet, dass** es sich bei der Hülle um ein flexibles Schlauchstück oder einen Ring handelt.

7. Verfahren nach einem der vorhergehenden Ansprüche 4 bis 6, **dadurch gekennzeichnet, dass** die Hülle aus einem Silikonelastomer gefertigt ist.

8. Medizinisches Gerät, insbesondere Dialysemaschine, umfassend
einen mechanischen Konnektor, der ein Paar an Verbindungsteilen (1, 4) aufweist, die korrespondierende Dichtflächen aufweisen, wobei
ein Verbindungsteil (1) einen männlichen Konus (2) und das andere Verbindungsteil (4) einen weiblichen Konus (5) aufweist, wobei wenigstens ein Abschnitt der äußeren Mantelfläche des männlichen Konus (2) und wenigstens ein Abschnitt der inneren Mantelfläche des weiblichen Konus (5) die Dichtflächen bilden, und
ein Zusammenführen der Dichtflächen dazu führt, dass der Kontakt der beiden Dichtflächen erhalten bleibt und die Dichtflächen mit einem gewissen Anpressdruck durch Verschrauben oder Verrasten aufeinanderliegen,
**dadurch gekennzeichnet, dass**
die Dichtflächen bei geschlossenem Konnektor zumindest abschnittsweise mit einer viskosen Flüssigkeit benetzt sind,
die viskose Flüssigkeit eine dynamische Viskosität von zwischen 10² und 10⁵ mPa·s bei 25°C aufweist, und
die viskose Flüssigkeit steril und biokompatibel ist.

9. Medizinisches Gerät nach Anspruch 8, wobei der Konnektor einen flüssigkeitsführenden Kreislauf, insbesondere einen extrakorporalen Blutkreislauf, mit einer Zu- oder Ableitung verbindet.

10. Medizinisches Gerät nach Anspruch 9, wobei der flüssigkeitsführende Kreislauf eine Pumpe umfasst, und der Konnektor auf einer Saugseite der Pumpe angeordnet ist.

11. Medizinisches Gerät nach einem der Ansprüche 8 bis 10, wobei der Verbindungsspalt zwischen den Verbindungsteilen (1, 4) des geschlossenen Konnektors mit einer Hülle abgedeckt ist.

12. Medizinisches Gerät nach einem der Ansprüche 8 bis 11, wobei es sich bei dem mechanischen Konnektor um einen Luer-Lock-Anschluss handelt.

## Claims

1. A method of increasing the air-tightness of a mechanical connector of a medical device, in particular of a dialysis machine, wherein
the mechanical connector has a pair of connection parts (1, 4) which have corresponding sealing surfaces,
one connection part (1) has a male cone (2) and the other connection part (4) has a female cone (5), with at least a portion of the outer lateral surface of the male cone (2) and at least a portion of the inner lateral surface of the female cone (5) forming the sealing surfaces, and
a joining together of the connection parts (1, 4) comprises the contact between the two sealing surfaces being maintained and the sealing surfaces lying against one another with a certain contact pressure by screwing or latching,
**characterised in that**
one of the two sealing surfaces is wetted with a viscous liquid before the connection parts (1, 4) are joined together,
the viscous liquid has a dynamic viscosity of between 10² and 10⁵ mPa·s at 25°C, and
the viscous liquid is sterile and biocompatible.

2. The method according to one of the preceding claims, **characterised in that** the mechanical connector is a Luer lock connector.

3. The method according to one of the preceding claims, **characterised in that** only the sealing surface of a connection part (1), preferably only the lateral surface of the male cone (2), is wetted with the viscous liquid before the connection parts (1, 4) are joined together.

4. The method according to one of the preceding claims, **characterised in that** the connection gap between the connection parts (1, 4) is covered by a sheath after they have been joined together.

5. The method according to claim 4, **characterised in that** the sheath is a flexible sheath, with provision preferably being made that this sleeve is slipped over the connector such that it completely covers the region of the connection gap.

6. The method according to one of the preceding claims 4 or 5, **characterised in that** the sheath is a flexible hose piece or a ring.

7. The method according to one of the preceding claims 4 to 6, **characterised in that** the sheath is made of a silicone elastomer.

8. A medical device, in particular a dialysis machine, comprising
a mechanical connector which has a pair of connection parts (1, 4) having corresponding sealing surfaces, wherein
one connection part (1) has a male cone (2) and the other connection part (4) has a female cone (5), with at least a portion of the outer lateral surface of the male cone (2) and at least a portion of the inner lateral surface of the female cone (5) forming the sealing surfaces,
a joining together of the sealing surfaces results in the contact between the two sealing surfaces being maintained and the sealing surfaces lying against one another with a certain contact pressure by screwing or latching,
**characterised in that**
the sealing surfaces are wetted at least in part with a viscous liquid when the connector is closed,
the viscous liquid has a dynamic viscosity of between 10² and 10⁵ mPa·s at 25°C, and
the viscous liquid is sterile and biocompatible.

9. The medical device according to claim 8, wherein the connector connects a liquid-conducting circuit, in particular an extracorporeal blood circuit, to an inflow or to an outflow.

10. The medical device according to claim 9, wherein the liquid-conducting circuit comprises a pump and the connector is arranged on an intake side of the pump.

11. The medical device according to one of claims 8 to 10, wherein the connection gap between the connection parts (1, 4) of the closed connector is covered by a sheath.

12. The medical device according to one of claims 8 to 11, wherein the mechanical connector is a Luer lock connector.

## Revendications

1. Procédé destiné à accroître l'étanchéité d'un connecteur mécanique d'un dispositif médical, en particulier d'une machine de dialyse,
le connecteur mécanique comportant une paire de pièces de raccordement (1, 4), qui comportent des surfaces d'étanchéité correspondantes,
une pièce de raccordement (1) comportant un cône mâle (2) et l'autre pièce de raccordement (4) un cône femelle (5), au moins une portion de la surface latérale extérieure du cône mâle (2) et au moins une portion de la surface latérale intérieure du cône femelle (5) formant les surfaces d'étanchéité, et
une réunion des pièces de raccordement (1, 4) comprenant le fait que le contact entre les deux surfaces d'étanchéité est conservé et que les surfaces d'étanchéité reposent l'une sur l'autre avec une certaine pression de serrage par vissage ou enclenchement,
**caractérisé en ce que**
avant la réunion des pièces de raccordement (1, 4), une des deux surfaces d'étanchéité est humectée avec un liquide visqueux,
le liquide visqueux présente une viscosité dynamique comprise entre 10² et 10⁵ mPa·s à 25 °C, et
le liquide visqueux est stérile et biocompatible.

2. Procédé selon l'une des revendications précédentes, **caractérisé en ce que** le connecteur mécanique est un raccord Luer Lock.

3. Procédé selon l'une des revendications précédentes, **caractérisé en ce que**, avant la réunion des pièces de raccordement (1, 4), seulement la surface d'étanchéité d'une pièce de raccordement (1), de préférence seulement la surface latérale du cône mâle (2) est humectée avec le liquide visqueux.

4. Procédé selon l'une des revendications précédentes, **caractérisé en ce que** le jeu de raccordement entre les pièces de raccordement (1, 4) est recouvert avec une gaine après leur réunion.

5. Procédé selon la revendication 4, **caractérisé en ce que** la gaine est une gaine flexible, cette gaine étant de préférence prévue pour être enfilée sur le connecteur de telle manière qu'elle recouvre entièrement la zone du jeu de raccordement.

6. Procédé selon l'une des revendications précédentes 4 ou 5, **caractérisé en ce que** la gaine est un morceau de tube flexible ou une bague.

7. Procédé selon l'une des revendications précédentes 4 à 6, **caractérisé en ce que** la gaine est fabriquée en élastomère de silicone.

8. Dispositif médical, en particulier machine de dialyse, comprenant
un connecteur mécanique qui comporte une paire de pièces de raccordement (1, 4), qui comportent des surfaces d'étanchéité correspondantes,
une pièce de raccordement (1) comportant un cône mâle (2) et l'autre pièce de raccordement (4) un cône femelle (5), au moins une portion de la surface latérale extérieure du cône mâle (2) et au moins une portion de la surface latérale intérieure du cône femelle (5) formant les surfaces d'étanchéité, et
une réunion des surfaces d'étanchéité ayant pour résultat que le contact entre les deux surfaces d'étanchéité est conservé et que les surfaces d'étanchéité reposent l'une sur l'autre avec une certaine pression de serrage par vissage ou enclenchement,
**caractérisé en ce que**
lorsque le connecteur est fermé, les surfaces d'étanchéité sont humectées au moins sur certaines parties avec un liquide visqueux,
le liquide visqueux présente une viscosité dynamique comprise entre 10² et 10⁵ mPa·s à 25 °C, et
le liquide visqueux est stérile et biocompatible.

9. Dispositif médical selon la revendication 8, dans lequel le connecteur relie un circuit véhiculant un liquide, en particulier un circuit de sang extracorporel, à une conduite d'arrivée ou d'évacuation.

10. Dispositif médical selon la revendication 9, dans lequel le circuit véhiculant un liquide comprend une pompe, et le connecteur est disposé sur un côté aspiration de la pompe.

11. Dispositif médical selon l'une des revendications 8 à 10, dans lequel le jeu de raccordement entre les pièces de raccordement (1, 4) du connecteur fermé est recouvert avec une gaine.

12. Dispositif médical selon l'une des revendications 8 à 11, dans lequel le connecteur mécanique est un raccord Luer Lock.
